# EUROPÄISCHE PATENTSCHRIFT

(11) **EP 1 062 961 B1**
(45) Veröffentlichungstag und Bekanntmachung des Hinweises auf die Patenterteilung: **27.07.2005**
(21) Anmeldenummer: 00104199.5
(22) Anmeldetag: 01.03.2000
(51) Int. Cl.: A61M 5/31, A61M 5/315

(54) **Spritze für medizinische Zwecke**
Medical syringe
Seringue médicale

(30) Priorität: 26.06.1999 DE 19929325
(43) Veröffentlichungstag der Anmeldung: 27.12.2000
(73) Patentinhaber: Arzneimittel GmbH Apotheker Vetter & Co. Ravensburg, D-88212 Ravensburg (DE)
(72) Erfinder: Vetter, Udo J., 88214 Ravensburg (DE); Glocker, Joachim, 88250 Weingarten (DE); Otto, Thomas, 88250 Weingarten (DE)
(74) Vertreter: Dziewior, Joachim, Dipl.-Phys. Dr.

(56) Entgegenhaltungen:
- US-A- 2 854 975
- US-A- 3 811 441
- US-A- 4 129 130
- US-A- 5 700 247

## Beschreibung

Die Erfindung betrifft eine Spritze für medizinische Zwecke, mit einem Spritzenzylinder und wenigstens einem darin verschiebbar angeordneten Spritzenkolben, wobei der Spritzenzylinder an seinem einen Ende mit einer Kanüle versehen ist oder versehen werden kann und an seinem anderen Ende eine Fingerauflage aufweist, die auf einen radial nach außen vorstehenden Rollrand des Spritzenzylinders aufrastbar ist, wobei die Fingerauflage von einer Fingerplatte gebildet ist, die einen randseitig angeschlossenen, ebenfalls zum kanülenseitigen Ende vorstehenden Kragen aufweist, wobei der Kragen an zwei der sich gegenüberstehenden Seiten der Fingerauflage einen Haltebügel bildet, wobei an jedem Haltebügel ein radial nach innen vorstehendes Rastelement angeordnet ist, das bei aufgesetzter Fingerauflage den Rollrand des Spritzenzylinders hintergreift.

Derartige Spritzen sind in vielfältigen Ausführungsformen in Gebrauch und beispielsweise in der DE 41 27 650 vorbeschrieben.

Aus der US-A-5 700 247 ist eine Spritze mit einer Fingerauflage bekannt, bei der die Fingerauflage aus einer Fingerplatte sowie einen daran angeschlossenen Mantelteil besteht. Das Mantelteil ist mit seinem Innendurchmesser an den Außendurchmesser des Spritzenzylinders angepasst; desweiteren weist das Mantelteil eine Ringnut auf, die den endseitig am Glaszylinder bestehenden Rollrand in sich aufnimmt.

Um die Fingerauflage am Spritzenzylinder anschließen zu können, muß das Mantelteil aufgespreizt werden können. Hierzu weisen die Fingerplatte sowie das Mantelteil einen axial verlaufenden Schlitz auf, durch den das Mantelteil in ausreichendem Maße aufspreizen kann, um den Spritzenzylinder in der Fingerauflage einrasten zu lassen.

Um ein unbeabsichtigtes Herausziehen des Stopfens aus dem Spritzenzylinder zu verhindern, ist der Durchmesser der Fingerplatte auf der der Stirnfläche des Spritzenzylinders anliegenden Seite etwas kleiner als der lichte Innendurchmesser des Spritzenzylinders, wodurch ein Anschlag für den Stopfen gebildet wird. Um gleichwohl die Kolbenstange mit Stopfen auch bei montierter Fingerauflage einsetzen zu können, weist die Fingerplatte eine sich zum Spritzenzylinder hin konisch verjüngende Öffnung auf.

Diese vorbekannte Fingerauflage besitzt, jedenfalls für das Aufsetzen auf den Spritzenzylinder, keine zentrierende Wirkung, so daß für die Montage entsprechende - aufwendige - Maßnahmen getroffen werden müssen.

Der Erfindung liegt die Aufgabe zugrunde, eine Spritze der eingangs genannten Art so auszubilden, daß die Fingerauflage auf möglichst einfache Weise montiert werden kann und zugleich eine Stopfenbremse bildet, wobei darüber hinaus die Möglichkeit bestehen soll, den Stopfen beim Zurückziehen der Kolbenstange an einer dem jeweiligen Spritzentyp entsprechend dem Anwendungsfall vorgesehenen Stelle stoppen zu können.

Diese Aufgabe wird nach der Erfindung dadurch gelöst, daß der Haltebügel von der Fingerplatte freigeschnitten ist, wobei die Fingerplatte im wesentlichen rechteckige Querschnittsgestalt besitzt und die Haltebügel U-förmig mit einem geradlinig verlaufenden Bügelrücken und jeweils nur kurzen Bügelschenkeln gestaltet sind, und daß die Fingerplatte einen ins Innere des Spritzenzylinders vorstehenden, zugleich ein Zentrierelement bildenden hülsenförmigen Ansatz aufweist.

Der durch die Erfindung erreichte Vorteil besteht im wesentlichen darin, daß je nach Wunsch bzw. Einsatz der Spritze eine Fingerauflage mit entsprechend langem hülsenförmigem Ansatz ausgewählt und verwendet werden kann. Somit wird vor der Injektion die Wegstrecke beim Zurückziehen der Kolbenstange - also zum Beispiel während des Aspirierens von Blut - durch das Anschlagen des Stopfens an dem hülsenförmigen Ansatz entsprechend begrenzt.

Weiter wird es durch die Erfindung möglich, eine vorgefüllte Spritze ohne Unterbrechung der Arbeitsfolge, also ohne Zwischenlagerung von vormontierten, halbfertigen Produkten, in einem kontinuierlichen Linienprozeß schnell zu fertigen. Darüber hinaus sind für die Montage nur wenige sich bewegende Teile notwendig, wodurch infolge der geringeren Abnutzung eine weitere Kostenersparnis erreicht wird. Durch das zentrische Aufsetzen der Fingerauflage auf den Spritzenzylinder, ist die Montage deutlich gegenüber solchen Systemen vereinfacht, bei denen die Fingerauflage seitlich über den Rollrand des Glaszylinders, also in radialer Richtung, geschoben werden muß.

Als vorteilhaft hat sich eine Ausgestaltung erwiesen, bei der der Außendurchmesser des hülsenförmigen Ansatzes etwa dem Innendurchmesser des Spritzenzylinders entspricht, also gleich oder allenfalls geringfügig kleiner ist.

Um bei der Montage eine noch bessere Zentrierung zu erreichen, empfiehlt es sich, daß die senkrecht zur Fingerplatte gemessene Höhe des hülsenförmigen Ansatzes größer ist als die Höhe des Kragens.

Um weiter eine drehfeste Montage der Fingerauflage am Spritzenzylinder zu erreichen, sieht die Erfindung vor, daß der Rollrand des Spritzenzylinders in Draufsicht eine im wesentlichen ovale Form hat, wobei das Rastelement den rund verlaufenden Teil des Rollrandes hintergreift.

Weiter ist es günstig, wenn der Rand der in die Fingerplatte mündenden Bohrung des hülsenförmigen Ansatzes auf der der Kanüle abgewandten Seite mit einer konischen Anlaufschräge versehen ist.

Schließlich ist es insbesondere fertigungstechnisch von Vorteil, wenn die Fingerplatte, der Kragen, die Haltebügel und der hülsenförmige Ansatz einstückig ausgebildet sind und aus relativ formsteifem Material bestehen. Dadurch ist einerseits eine gute Handhabbarkeit der Spritze sichergestellt, andererseits besitzen die Haltebügel hierdurch hinreichende Formsteifigkeit, um ein selbsttätiges Lösen der Fingerauflage vom Spritzenzylinder zu verhindern.

Im folgenden wird die Erfindung an einem in der Zeichnung dargestellten Ausführungsbeispiel näher erläutert; es zeigen:
- Fig. 1: eine nur teilweise dargestellte Spritze mit aufgesetzter Fingerauflage,
- Fig. 2: den Gegenstand nach Fig. 1, jedoch in demgegenüber rückwärtiger Ansicht,

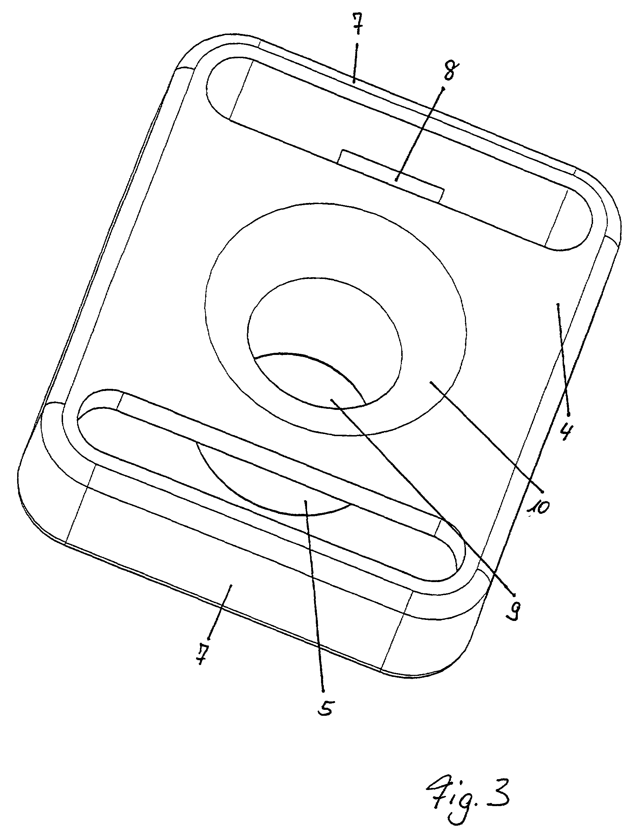

- Fig. 3: den Gegenstand nach Fig. 1, jedoch ohne Spritzenzylinder,
- Fig. 4: den Gegenstand nach Fig. 2, jedoch ohne Spritzenzylinder,
- Fig. 5: eine Montagemöglichkeit für die Spritze bei schräg angelieferten Fingerauflagen.

Die in der Zeichnung dargestellte und für medizinische Zwecke vorgesehene Spritze besteht aus einem nur teilweise dargestellten Spritzenzylinder 1, an dessen nicht wiedergegebenen Ende eine Kanüle angeordnet sein bzw. werden kann. Ebenfalls nicht dargestellt ist der im Spritzenzylinder 1 üblicherweise verschiebbar angeordnete Spritzenkolben sowie die zu seiner Verschiebung benötigte Kolbenstange.

An dem Spritzenzylinder 1 ist eine Fingerauflage 2 angeschlossen, die auf einen radial nach außen vorstehenden Rollrand 3 des Spritzenzylinders 1 aufgerastet ist.

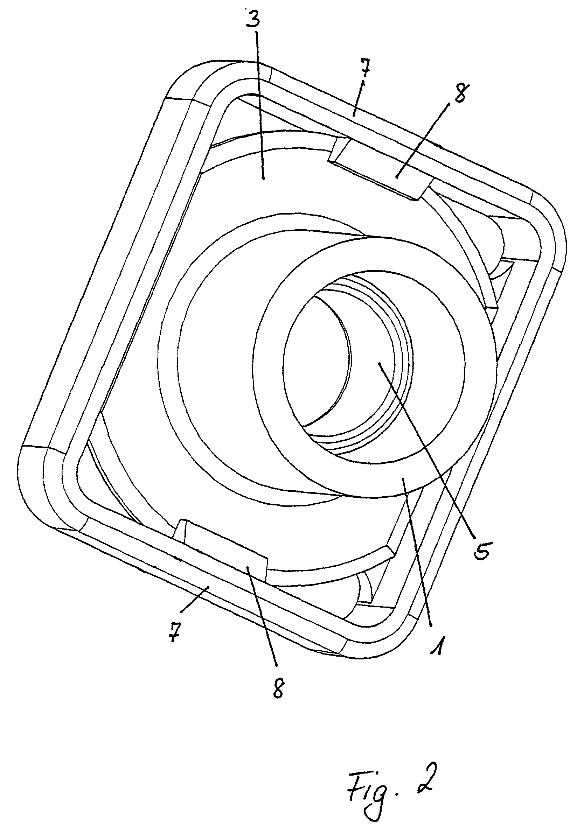

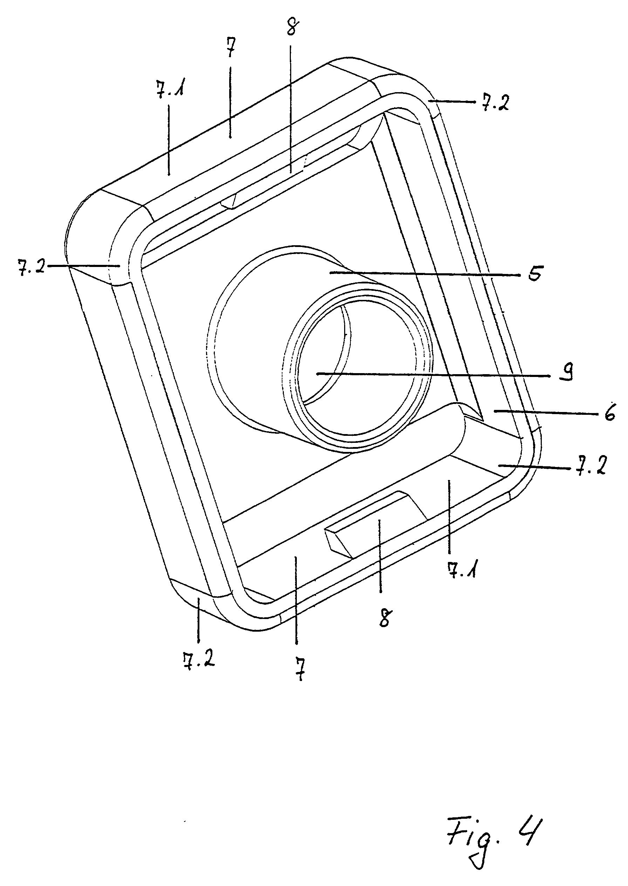

Im einzelnen besteht die Fingerauflage 2 aus einer Fingerplatte 4, die einen ins Innere des Spritzenzylinders 1 vorstehenden, hülsenförmigen Ansatz 5 aufweist. Dieser ist insbesondere in der Fig. 4 deutlich zu sehen. Weiter ist an der Fingerplatte 4 randseitig ein ebenfalls zum kanülenseitigen Ende vorstehender Kragen 6 angeschlossen, der an zwei der sich gegenüberstehenden Seiten der Fingerauflage 2 von der Fingerplatte 4 freigeschnitten ist. Auf diese Weise werden zwei Haltebügel 7 gebildet, wobei an jedem Haltebügel 7 ein radial nach innen stehendes Rastelement 8 angeordnet ist. Dieses Rastelement 8 hintergreift, wie sich insbesondere aus der Fig. 2 ergibt, den Rollrand 3 des Spritzenzylinders 1.

Die Fingerplatte 4 besitzt eine im wesentlichen rechteckige Querschnittsgestalt, während die Haltebügel 7 etwa U!förmig gestaltet sind. Dabei weisen die Haltebügel 7 einen geradlinig verlaufenden Bügelrücken 7.1 und jeweils nur kurze Bügelschenkel 7.2 auf. Der Übergangsbereich vom Bügelrücken 7.1 zum Bügelschenkel 7.2 ist verrundet, wodurch sich die Handhabung der Spritze angenehmer gestaltet.

Der Außendurchmesser des hülsenförmigen Ansatzes 5 entspricht etwa dem Innendurchmesser des Spritzenzylinders 1, liegt also diesem allenfalls mit minimalem Spalt an. Die senkrecht zur Fingerplatte 4 gemessene Höhe des hülsenförmigen Ansatzes 5 ist wenigstens geringfügig höher als die Höhe des Kragens 6, wodurch der hülsenförmige Ansatz 5 eine Zentrierhilfe beim Aufsetzen der Fingerauflage 2 auf den Spritzenzylinder 1 bildet. Die tatsächliche Höhe des hülsenförmigen Ansatzes 5 wird jedoch bestimmt durch dessen zweite Funktion, nämlich eine Bremse für den Stopfen zu bilden, wobei die Höhe des Ansatzes 5 die Stelle bestimmt, über die hinaus der Stopfen nicht in rückwärtiger Richtung verstellt werden soll.

Wie sich insbesondere aus der Fig. 2 ersehen läßt, hat der Rollrand 3 des Spritzenzylinders 1 in Draufsicht eine im wesentlichen ovale Form, wobei das Rastelement 8 den rund verlaufenden Teil des Rollrandes 3 hintergreift. Diese ovale Form ergibt sich am einfachsten durch Abschneiden des Rollrandes 3 auf zwei sich gegenüberstehenden Seiten.

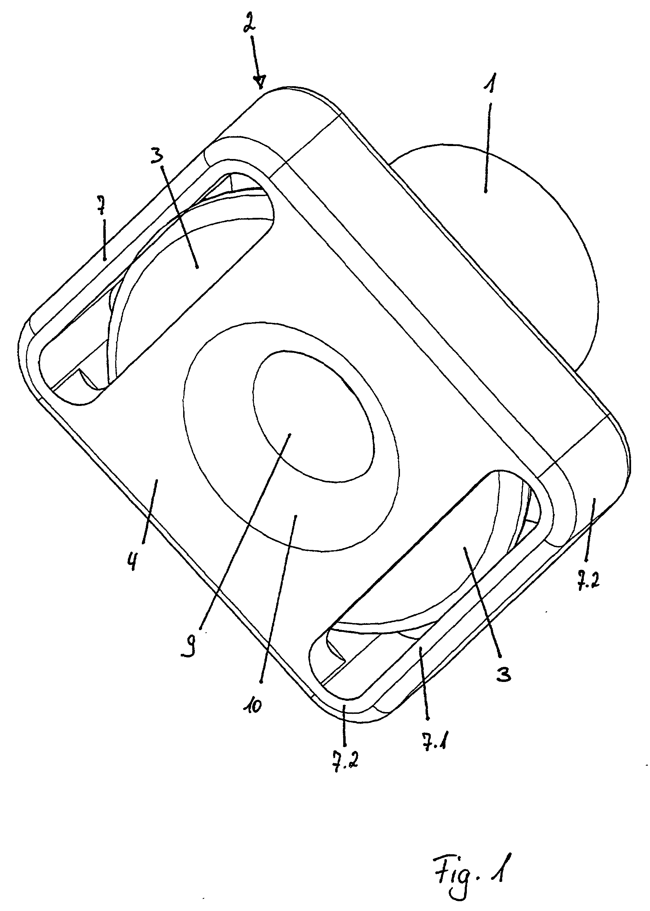

Wie sich aus Fig. 1 ersehen läßt, ist der Rand der in die Fingerplatte 4 mündenden Bohrung 9 des hülsenförmigen Ansatzes 5 auf der der Kanüle abgewandten Seite mit einer konischen Anlaufschräge 10 versehen.

Schließlich sind die Fingerplatte 4, der Kragen 6, der Haltebügel 7 und der hülsenförmige Ansatz 5 einstückig ausgebildet und bestehen aus relativ formsteifem Material, wodurch eine sichere Handhabung der Spritze beim Gebrauch gewährleistet und im übrigen sichergestellt ist, daß die Fingerauflage 2 sich nicht selbsttätig vom Spritzenzylinder 1 lösen kann.

Im Produktionsablauf der Spritze erfolgt zunächst die Füllung des Spritzenzylinders 1 von der zum Anschluß der Fingerauflage 2 vorgesehenen Seite her, worauf der Spritzenzylinder 1 durch Setzen des Spritzenkolbens geschlossen wird. Die vorgefüllte Spritze wird im Anschluß daran durch Montage der Fingerauflage 2 vervollständigt. Hierfür bieten sich zwei unterschiedliche Möglichkeiten an:

Bei einer Montage in axialer Richtung wird die Fingerauflage 2 zunächst so auf den Rollrand 3 des Glaszylinders aufgesetzt, daß der hülsenförmige Ansatz 5 vom Spritzenzylinder 1 zentrisch aufgenommen wird. Anschließend wird die Fingerauflage 2 mit definiertem, axialem Druck gegen den Klemmwiderstand der über die Haltebügel 7 elastisch nachgiebigen Rastelemente 8 soweit über den Rollrand 3 geschoben, bis diese (hörbar) hinter dem Rollrand 3 einrasten.

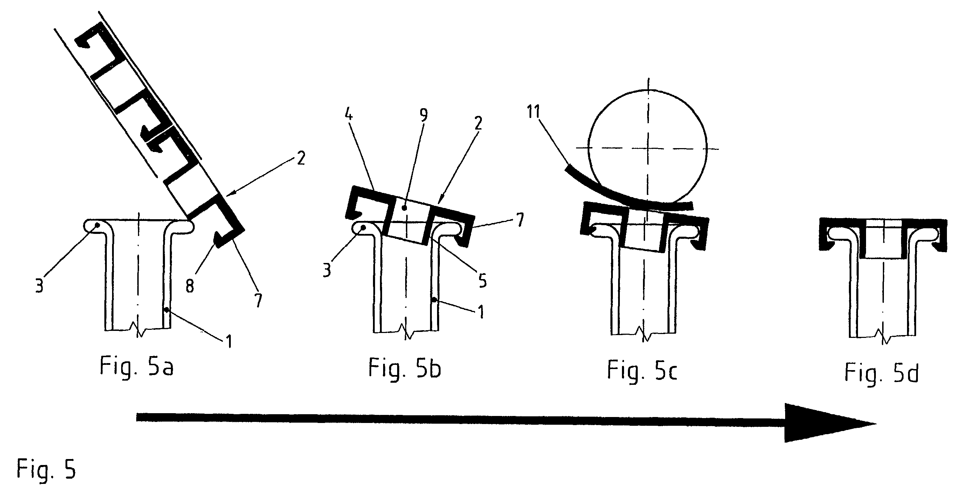

Es besteht jedoch auch die in Fig. 5 dargestellte Möglichkeit, die Fingerauflage 2 schräg (z. B. unter einem Winkel von ca. 45 Grad zum Spritzenzylinder) beizustellen, so daß zunächst eines der beiden Rastelemente 8 den Rollrand 3 untergreift, wie dies in Fig. 5b dargestellt ist. Auf diese Weise wird die Fingerauflage 2 bereits mit dem einen Rastelement 8 am Spritzenzylinder 1 arretiert. Beim Weitertransport des Spritzenzylinders 1 und gleichzeitiger Mitnahme der Fingerauflage 2 durchläuft die Spritze einen Niederhalter 11, der beispielsweise kreisrunde oder auch sichelförmige Gestalt aufweisen kann. Dies ist in Fig. 5c schematisch dargestellt. Dieser Niederhalter 11 bewirkt das vollständige Aufdrücken der Fingerauflage 2, also auch das Einrasten des zweiten Rastelementes 8, wie sich dies aus Fig. 5d ergibt.

## Patentansprüche

1. Spritze für medizinische Zwecke, mit einem Spritzenzylinder (1) und wenigstens einem darin verschiebbar angeordneten Spritzenkolben, wobei der Spritzenzylinder (1) an seinem einen Ende mit einer Kanüle versehen ist oder versehen werden kann und an seinem anderen Ende eine Fingerauflage (2) aufweist, die auf einen radial nach außen vorstehenden Rollrand (3) des Spritzenzylinders (1) aufrastbar ist, wobei die Fingerauflage (2) von einer Fingerplatte (4) gebildet ist, die einen randseitig angeschlossenen, ebenfalls zum kanülenseitigen Ende vorstehenden Kragen (6) aufweist, wobei der Kragen (6) an zwei der sich gegenüberstehenden Seiten der Fingerauflage (2) einen Haltebügel (7) bildet, wobei an jedem Haltebügel (7) ein radial nach innen vorstehendes Rastelement (8) angeordnet ist, das bei aufgesetzter Fingerauflage (2) den Rollrand (3) des Spritzenzylinders (1) hintergreift, **dadurch gekennzeichnet, daß** der Haltebügel (7) von der Fingerplatte (4) freigeschnitten ist, wobei die Fingerplatte (4) im wesentlichen rechteckige Querschnittsgestalt besitzt und die Haltebügel (7) U!förmig mit einem geradlinig verlaufenden Bügelrücken (7.1) und jeweils nur kurzen Bügelschenkeln (7.2) gestaltet sind, und daß die Fingerplatte (4) einen ins Innere des Spritzenzylinders (1) vorstehenden, zugleich ein Zentrierelement bildenden hülsenförmigen Ansatz (5) aufweist.

2. Spritze nach Anspruch 1, **dadurch gekennzeichnet, daß** der Übergangsbereich vom Bügelrücken (7.1) zum Bügelschenkel (7.2) verrundet ist.

3. Spritze nach Anspruch 1 oder 2, **dadurch gekennzeichnet, daß** der Außendurchmesser des hülsenförmigen Ansatzes (5) etwa dem Innendurchmesser des Spritzenzylinders (1) entspricht.

4. Spritze nach einem der Ansprüche 1 bis 3, **dadurch gekennzeichnet, daß** die senkrecht zur Fingerplatte (4) gemessene Höhe des hülsenförmigen Ansatzes (5) größer ist als die Höhe des Kragens (6).

5. Spritze nach einem der Ansprüche 1 bis 4, **dadurch gekennzeichnet, daß** der Rollrand (3) des Spritzenzylinders (1) in Draufsicht eine im wesentlichen ovale Form hat, wobei das Rastelement (8) den rund verlaufenden Teil des Rollrandes (3) hintergreift.

6. Spritze nach einem der Ansprüche 1 bis 5, **dadurch gekennzeichnet, daß** der Rand der in die Fingerplatte (4) mündenden Bohrung (9) des hülsenförmigen Ansatzes (5) auf der der Kanüle abgewandten Seite mit einer konischen Anlaufschräge (10) versehen ist.

7. Spritze nach einem der Ansprüche 1 bis 6, **dadurch gekennzeichnet, daß** die Fingerplatte (4), der Kragen (6), die Haltebügel (7) und der hülsenförmige Ansatz (5) einstückig ausgebildet sind und aus relativ formsteifem Material bestehen.

## Claims

1. Syringe for medical purposes, with a syringe barrel (1) and at least one syringe plunger arranged within it, wherein at one end the syringe barrel (1) is equipped or can be equipped with a cannula, and at the other end it has a finger rest (2) that can be snapped onto a rolled edge (3), projecting radially outwards, of the syringe barrel (1), wherein the finger rest (2) is formed by a finger plate (4) that has collar (6) which is connected at the edge and which likewise projects towards that end bearing the cannula, wherein on two of the opposing sides of the finger rest (2) the collar (6) forms a holding bar (7), wherein arranged on each holding bar (7) is a catch element (8) which projects inwards and which engages behind the rolled edge (3) of the syringe barrel (1) when the finger rest (2) is fitted, **characterised in that** the holding bar (7) is cut away from the finger plate (4), wherein the finger plate (4) has a cross-sectional shape that is essentially rectangular and the holding bar (7) is U-shaped with a bar spine (7.1) that runs in a straight line and the bar sides (7.2) are respectively designed to be only short, and that the finger plate (4) has a sleeve-shaped extension piece (5) that projects into the interior of the syringe barrel (1) and simultaneous forms a centring element.

2. Syringe in accordance with claim 1, **characterised in that** the transitional area from the bar spine (7.1) to the bar side (7.2) is rounded.

3. Syringe in accordance with claim 1 or 2, **characterised in that** the external diameter of the sleeve-shaped extension piece (5) corresponds approximately to the internal diameter of the syringe barrel (1).

4. Syringe in accordance with one of the claims 1 to 3, **characterised in that** the height of the sleeve-shaped extension piece (5) as measured perpendicular to the finger plate (4) is greater than the height of the collar (6).

5. Syringe in accordance with one of the claims 1 to 4, **characterised in that** in top view, the rolled edge (3) of the syringe barrel (1) has an essentially oval shape, wherein the catch element (8) engages behind that part of the rolled edge (3) that runs in a circular curve.

6. Syringe in accordance with one of the claims 1 to 5, **characterised in that** on the side facing away from the cannula, that edge of the hole (9) of the sleeve-shaped extension piece (5) which opens into the finger plate (4) is equipped with a conical approach slope (10).

7. Syringe in accordance with one of the claims I to 6, **characterised in that** the finger plate (4), the collar (6), the holding bar (7) and the sleeve-shaped extension piece (5) are designed in one piece and comprise relatively dimensionally stable material.

## Revendications

1. Seringue à usage médical comprenant un cylindre de seringue (1) avec au moins un piston de seringue coulissant dans ce cylindre qui, à une extrémité est équipé ou peut l'être, d'une canule, tandis qu'à son autre extrémité il présente un appui de doigts (2) qui peut être encliqueté sur le bord roulé (3) faisant saillie radialement vers l'extérieur, du cylindre de seringue (1), cet appui de doigts (2) étant constitué d'une plaque de doigts (4) qui présente, raccordé à son bord, un collet (6) également en saillie en direction de l'extrémité portant la canule, et formant le long de deux côtés opposés de l'appui de doigts (2) un étrier de maintien (7) sur lequel est monté en saillie radialement vers l'extérieur un élément d'encliquetage (8) qui est en prise par derrière avec le bord roulé (3) du cylindre de seringue (1) quand l'appui de doigts (2) est monté, **caractérisée en ce que** l'étrier de maintien (7) est réalisé par découpe de la plaque de doigts (4) qui présente en section une forme essentiellement rectangulaire, tandis que l'étrier de maintien (7) a la forme d'un U avec un dos (7.1) rectiligne encadré de branches d'étrier (7.2) courtes, la plaque de doigts (4) présentant un appendice (5) en forme de douille qui fait saillie vers l'intérieur du cylindre de seringue (1) et qui constitue en même temps un élément de centrage.

2. Seringue selon la revendication 1, **caractérisée en ce que** la zone de passage du dos d'étrier (7.1) à la branche d'étrier (7.2) est arrondie.

3. Seringue selon la revendication 1 ou 2, **caractérisée en ce que** le diamètre externe de l'appendice (5) en forme de douille correspond à peu près au diamètre interne du cylindre de seringue (1).

4. Seringue selon une des revendications 1 à 3, **caractérisée en ce que** la hauteur de l'appendice (5) en forme de douille, mesurée perpendiculairement à la plaque de doigts (4), est supérieure à la hauteur du collet (6).

5. Seringue selon une des revendications 1 à 4, **caractérisée en ce que** le bord roulé (3) du cylindre de seringue (1) a en plan une forme essentiellement ovale, et que l'élément d'encliquetage (8) est en prise derrière la partie arrondie du bord roulé (3).

6. Seringue selon une des revendications 1 à 5, **caractérisée en ce que** le bord de l'alésage (9) de l'appendice (5) en forme de douille qui débouche dans la plaque de doigts (4) présente, sur son côté éloigné de la canule, une pente d'engagement (10) conique.

7. Seringue selon une des revendications 1 à 6, **caractérisée en ce que** la plaque de doigts (4), le collet (6), l'étrier de maintien (7) et l'appendice (5) en forme de douille forment une seule pièce faite d'un matériau présentant une certaine rigidité de forme.
